# EUROPEAN PATENT APPLICATION

(11) **EP 2 614 768 A1**
(43) Date of publication of application: **17.07.2013**
(21) Application number: 13150950.7
(22) Date of filing: 11.01.2013
(51) Int. Cl.: A61B 1/24, A61B 5/00, A61B 1/00

(54) **System and method for three-dimensional intra-oral imaging**

(30) Priority: 13.01.2012 US 201213350629
(71) Applicant: ORMCO CORPORATION, Orange, CA 92867 (US)
(72) Inventor: Dillon, Robert, F, Bedford, New Hampshire 03110 (US); Gant, Evan, I.T., Medford, Massachusetts 02155 (US); Sickles, James, A, Mission Viejo, California 92691 (US); Leung, Philip, C.Y., Waltham, Massachusetts 02453 (US); Andreiko, Craig, A., Alta Loma, California 91737 (US)
(74) Representative: Findlay, Alice Rosemary

(57) **Abstract**

A portable three-dimensional intra-oral imaging system (102) is comprised of a processor (114), a housing (200) encasing the processor, an imaging sensor (108) extensibly coupled to the housing for capturing images, and a touchscreen display (116) to display the images acquired by the imaging sensor. An arm (702) is coupled to the housing and supports the touchscreen display, wherein the arm is configurable to be retracted and protracted to allow placement of the touchscreen display within a range of heights. A handle (128) of the housing is provided for portability.

## Description

The disclosure relates to a system and method for three-dimensional (3D) intra-oral imaging.

An intra-oral optical impression system is diagnostic equipment that allows a dental practitioner to see the inside of patient's mouth and display the topographical characteristics of teeth on a display monitor. Certain 3D intra-oral imagers may be comprised of an intra-oral camera with a light source. The 3D intra-oral imager may be inserted into the oral cavity of a patient by a dental practitioner. After insertion of the intra-oral imager into the oral cavity, the dental practitioner may capture images of visible parts of the teeth and the gingivae. 3D intra-oral imagers may be used to replace traditional cast impressions that record dental and orthodontic features.

The 3D intra-oral imager may be fabricated in the form of a slender rod that is referred to as a wand or a handpiece. The wand may be approximately the size of a dental mirror with a handle that is used in dentistry. The wand may have a built-in light source and a video camera that may achieve an imaging magnification, ranging in scale from 1 to 40 times or more. This allows the dental practitioner to discover certain types of details and defects of the teeth and gums. The images captured by the intra-oral camera may be displayed on a television or a computer monitor.

The wand may be attached or linked to a computer and a display monitor. The wand, the computer, and the display monitor may all be placed in a cart that is wheeled to the proximity of the patient before the dental practitioner places the tip of the wand inside the oral cavity of the patient and starts acquiring images. The acquired images may be displayed on the display monitor and may also be saved on a storage device. Furthermore, the acquired images may be transmitted to a remote computational device for additional processing. Such 3D intra-oral imager configurations may be inconvenient to move from one place to another because of the size and weight of the computer and the display monitor associated with the wand.

Provided are a method and a system for three-dimensional intra-oral imaging. A portable three-dimensional intra-oral imaging system is comprised of a processor, a housing encasing the processor, an imaging sensor extensibly coupled to the housing for capturing images, and a touchscreen display to display the images acquired by the imaging sensor. An arm is coupled to the housing and supports the touchscreen display, wherein the arm is configurable to be retracted and protracted to allow placement of the touchscreen display within a range of heights. A handle of the housing is provided for portability.

In certain embodiments, the handle is integral to the housing, and in other embodiments the handle is hinged to the housing.

In certain embodiments, the portable 3D intra-oral imaging system further comprises a wand, and a wand storage area. The wand comprises a proximal end and a distal end. The proximal end is extensibly attached to the housing by a retractable or stowable cord. The distal end has a tip that holds a disposable mirror. The wand storage area comprises at least one surface to protect the tip of the wand, wherein the wand storage area engages the wand with one or more fastners that grip the wand and one or more projections that fit into the wand.

In additional embodiments, one or more controls are located on the wand to start and stop recording the images and to navigate graphical user interface items displayed on the touchscreen display.

In yet additional embodiments, the tip is at least partially covered with a disposable sheath, and a disposable mirror is placed on or in the tip.

In further embodiments, the portable 3D intra-oral imaging system further comprises a health check artifact located on the housing, wherein in response to the wand being fully engaged with the one or more fasteners and the one or more projections, the tip of wand is aligned with the health check artifact, and wherein the health check artifact is configured to determine whether the disposable mirror placed on the tip of the wand is clean or otherwise satisfactory, and to determine whether the measurement accuracy of the three-dimensional imaging system exceeds a predetermined threshold.

In further embodiments, the stowable cord is wrapped around a cord wrap mechanism in response to the wand being placed on the wand storage area, wherein the handle does not touch the retractable cord.

In certain embodiments, the housing has a bottom surface with a front end and a back end. A plurality of pegs are mounted at the bottom surface of the housing, wherein the plurality of pegs are fabricated out of rubber or other non-slip material to prevent slippage.

In certain embodiments, a plurality of casters are mounted at the bottom surface of the housing to facilitate mobility.

In further embodiments, two pegs are mounted at the bottom surface towards the front end to provide stability and prevent slippage, and two casters are mounted at the bottom surface towards the back end to facilitate mobility.

In certain embodiments, the portable 3D intra-oral imaging system further comprises a battery driven power supply enclosed in the housing.

In further embodiments, the housing further comprises a bottom panel with a first set of ventilating holes, and a back panel with a second set of ventilating holes, wherein the first and the second set of ventilating holes allow circulation of air and dissipation of heat, and wherein at least one panel is removable to allow access to the battery driven power supply enclosed in the housing.

In yet further embodiments, the arm is rotatable and the touchscreen display is tiltable. The arm includes a spring counterbalancing mechanism to lift the touchscreen display. Additionally, the arm includes a friction bearing mechanism to keep the touchscreen display in a stationary position, in response to the touchscreen display being lifted.

In additional embodiments, the portable 3D intra-oral imaging system is not attached to any keyboard or mouse, and the portable 3D intra-oral imaging system further comprises a hard disk or a solid state memory device protectively encased within the housing to store the images acquired by the imaging sensor.

In further embodiments, the touchscreen display is disposed in a display enclosure, wherein a recessed area in the display enclosure assists a user to pull the touchscreen display out of a stored position into an upright position. Additionally, another recessed area in the display enclosure allows the arm to be stored flush to the housing.

Provided further is a method in which a 3D intra-oral imaging system is carried from one location to another via a handle coupled to a housing of the 3D intra-oral imaging system. An arm coupled to the touchscreen display is protracted to adjust the touchscreen display to a desired height over the housing. Images are acquired via an imaging sensor extensibly coupled to the housing. A processor encased within the housing processes the acquired images, and displays the processed images on the touchscreen display.

In further embodiments, a wand holding the imaging sensor is removed from a wand storage area located on the housing, prior to the acquiring of the images, wherein the wand comprises a proximal end and a distal end. The proximal end is extensibly attached to the housing by a stowable cord. The distal end has a tip that holds a disposable mirror. The wand storage area comprises at least one surface to protect the tip, wherein the wand storage area is configurable to engage the wand with one or more fasteners that grip the wand and one or more projections that fit into the wand.

In yet further embodiments, one or more controls are located on the wand to start and stop recording the images and to navigate graphical user interface items displayed on the touchscreen display.

In additional embodiments, a determination is made that acquisition of images is completed. The arm is retracted to lower the touchscreen display, in response to determining that the arm was protracted. The wand is stored in the wand storage area. The disposable mirror and the disposable sheath on the tip of the wand are replaced.

In yet additional embodiments, a determination is made via a health check artifact located on the housing, whether the disposable mirror placed on the tip of the wand is clean or otherwise satisfactory. In response to determining that the disposable mirror placed on the tip of the wand is not clean or otherwise satisfactory, the disposable mirror and the disposable sheath on the tip of the wand are replaced.

The invention will now be further described by way of example with reference to the accompanying drawings, in which:

FIG. 1 illustrates a block diagram of a computing and imaging environment that at least includes a 3D intra-oral imaging system, in accordance with certain embodiments;

FIG. 2 illustrates a front view of the intra-oral imaging system, in accordance with certain embodiments;

FIG. 3 illustrates a side view of the intra-oral imaging system to show rotations of an exemplary handle, in accordance with certain embodiments;

FIG. 4 illustrates a wand and a wand storage area of the intra-oral imaging system, in accordance with certain embodiments;

FIG. 5 illustrates a health check artifact of the intra-oral imaging system, in accordance with certain embodiments;

FIG. 6 illustrates an area for coupling an arm that supports a touchscreen monitor of the intra-oral imaging system, in accordance with certain embodiments;

FIG. 7 illustrates an arm that couples a touchscreen display to the housing of the intra-oral imaging system, in accordance with certain embodiments;

FIG. 8 illustrates a display enclosure for the touchscreen monitor of the intra-oral imaging system, in accordance with certain embodiments;

FIG. 9 illustrates venting areas in the intra-oral imaging system, in accordance with certain embodiments;

FIG. 10 illustrates how a cord reel and an exemplary handle are implemented in the intra-oral imaging system, in accordance with certain embodiments;

FIG. 11 illustrates at least a back panel of the intra-oral imaging system, in accordance with certain embodiments;

FIG. 12 illustrates at least the front, left, right, and bottom panels of the intra-oral imaging system, in accordance with certain embodiments;

FIG. 13 illustrates how a wand is aligned with respect to the wand storage area of the intra-oral imaging system, in accordance with certain embodiments;

FIG. 14 illustrates an arm, and the front and back of the display enclosure of the intra-oral imaging system, in accordance with certain embodiments;

FIG. 15 illustrates a block diagram of software components that comprise an image acquisition and control application implemented in the intra-oral imaging system, in accordance with certain embodiments;

FIG. 16 illustrates a block diagram of certain operations performed with respect to the intra-oral imaging system, in accordance with certain embodiments; and

FIG. 17 illustrates a block diagram of a computational system that shows certain elements of the intra-oral imaging system, in accordance with certain embodiments.

Provided are certain embodiments of a small form factor portable 3D intra-oral imaging system that comprises an optical impression system to record the topographical characteristics of teeth. The 3D intra-oral imaging system may be used for various applications in dentistry, including the production of orthodontic appliances and accessories. The support electronics and computational hardware for the intra-oral imaging system are housed in a portable enclosure, in a manner such that a cart is not needed to move the intra-oral imaging system from one location to another. The lightweight nature of the 3D intra-oral imaging system allows movement of the intra-oral imaging system via a carrying handle. In certain embodiments, the carrying handle may be integral to the portable enclosure. In certain other embodiments, the carrying handle may be coupled via a hinge to the portable enclosure. The carrying handle may be implemented in different ways in different embodiments.

The 3D intra-oral imaging system is made compact and portable by design elements, such as, a carrying handle integral to the portable enclosure or a hinged and rotatable carrying handle, a lightweight touchscreen display whose height may be adjusted by retracting or protracting an arm that supports the touchscreen display, an avoidance of any keyboards or mouse, graphical user interface navigation controls present on the wand of the 3D intra-oral imaging system, a wand storage area for the wand, etc.

In certain embodiments, the tip of the wand is at least partially covered with a disposable molded plastic sheath and a disposable mirror and a health check artifact may be activated to determine whether the disposable molded plastic sheath is new and whether the disposable mirror is clean or otherwise satisfactory.

### Exemplary embodiments

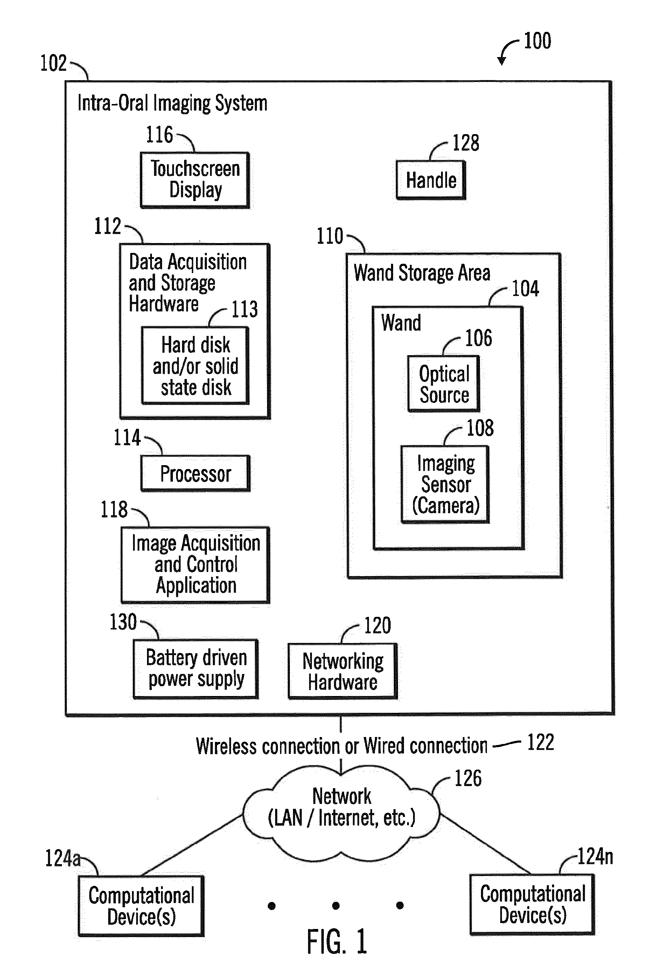

FIG. 1 illustrates a block diagram of a computing and imaging environment 100 that at least includes a 3D intra-oral imaging system 102 implemented in a compact and portable form factor, in accordance with certain embodiments. The intra-oral imaging system 102 is easily transportable from one place to another, and may comprise a special purpose computational device. In certain embodiments, the 3D intra-oral imaging system 102 may be referred to as a portable 3D intra-oral imaging system.

The 3D intra-oral imaging system 102 may include a wand 104 having an optical source 106 and an imaging sensor, such as, a camera 108. The optical source for wand illumination is designed to meet laser safety limits for visible light. The wand 104 includes appropriate eye safety warning labels. A dental practitioner may hold the wand 104 inside a patient's oral cavity to digitally capture the three-dimensional topography of the patient's teeth, gingivae, and/or palate. The wand is small and light weight for use by dental practitioners, and the imaging process is fast and simple to use, allowing the imaging of both arches and bites to be accomplished rapidly, such that a digital model of the imaged areas may be viewed on a touchscreen display 116.

In certain embodiments, the 3D intra-oral imaging system 102 is a precision opto-mechanical device, based on accordion fringe interferometer (AFI) techniques that measure three dimensional points on an object surface. The AFI technology projects two coherent beams of light, which create a precision interference fringe pattern visible on an object surface. In a simple single channel AFI system, a series of three two dimensional fringe images are acquired by an off-axis camera, with the fringe pattern at 0°, +120°, and -120°. For each camera pixel, the relative intensities of the three measurements may be mathematically combined to calculate a unique distance to the object surface, with the measurement limited by the width of one fringe. A multi-channel AFI system is not limited to a measurement width of one fringe, and is, therefore, capable of rapidly measuring the topology of intra-oral features.

The 3D intra-oral imaging system 102 may include a wand storage area 110 in which the wand 104 may be stored. The wand 104 may be coupled via a cord to a data acquisition and storage hardware 112 that is coupled to a processor 114 via circuitry. The data acquisition and storage hardware 112 may include a hard disk and/or solid state disk 113 that stores images acquired via the camera 108. The data acquisition and storage hardware 112 captures images acquired via the camera 108 of the wand 104 and the images may be processed by the processor 114 for display on the touchscreen display 116. The acquired images may be stored on shock resistant hard disks and/or solid state disks 113 in the 3D intra-oral imaging system 102.

An image acquisition and control application 118 implemented in hardware, software, firmware or any combination thereof may execute in the 3D intra-oral imaging system 102 and may perform operations that are executed via the processor 114. The image acquisition and control application 118 may display a graphical user interface on the touchscreen display 116, wherein various indicators displayed via graphical user interface elements, such as, icons, selections, drop down lists, radio buttons, lists, etc., may allow the dental practitioner to perform various operations with the 3D intra-oral imaging system 102 by touching the screen. There is no need for any keyboard, mouse, or trackball for interacting with the graphical user interface on the touchscreen display 116, and as a result the size and weight of the 3D intra-oral imaging system 102 is decreased in comparison to systems that do not utilize an integrated touchscreen display.

The operation of the wand 104 is controlled by the image acquisition and control application 118. The dental practitioner uses the graphical user interface to control the imaging process and may upload data to a web portal over the network 126. The graphical user interface displayed by the image acquisition and control application 118 may allow interactions with one or more of any suitable orthodontic practice management software packages, and may allow the sending of imagery data to other sites for further processing. For example, in certain embodiments, an orthodontic practitioner is able to choose from various settings in the graphical user interface to customize the imaging based upon the type of treatment desired or specific characteristics within the anatomy or existing dental appliances in the patient's mouth.

In certain embodiments, the 3D intra-oral imaging system 102 may include networking hardware 120 and associated networking software to connect via a wireless or a wired connection 122 to other computational devices 124a....124n over the network 126. The computational devices 124a...124n may include any suitable computational device such as a personal computer, a server computer, a mini computer, a mainframe computer, a blade computer, a tablet computer, a touchscreen computing device, a telephony device, a cell phone, a mobile computational device, etc., and some of the computational devices may provide web services or cloud computing services. The network 126 may comprise any suitable network know in the art such as a local area network, an intranet, the Internet, a storage area network, etc. In certain embodiments, after completion of the imaging procedure, the dental practitioner may be able to transfer the data file generated by the 3D intra-oral imaging system 102 in a manner compliant with government regulations and in a format that supports the manufacture of orthodontic and dental appliances, or transfer the data file for other applications.

The 3D intra-oral imaging system 102 may include a handle 128 that may be used for carrying the intra-oral imaging system 102 from one location to another. The handle 128 may also be referred to as a carrying handle. The intra-oral imaging system 102 also has a battery driven power supply 130 to maintain the operating system and other software while moving the intra-oral imaging system 102 from one place to another, after unplugging the intra-oral imaging system from direct electric power. The intra-oral imaging system 102 is designed such that the power requirements are relatively low and is less than 150 Watts.

In certain embodiments the 3D intra-oral imaging system 102 may report voids (holes) in the captured imaging surface data, and alert the dental practitioner to augment the data by using visual cues displayed on the touchscreen display 116. The intra-oral imaging system 102 also incorporates a system check to insure that the wand 104 is in proper operational condition.

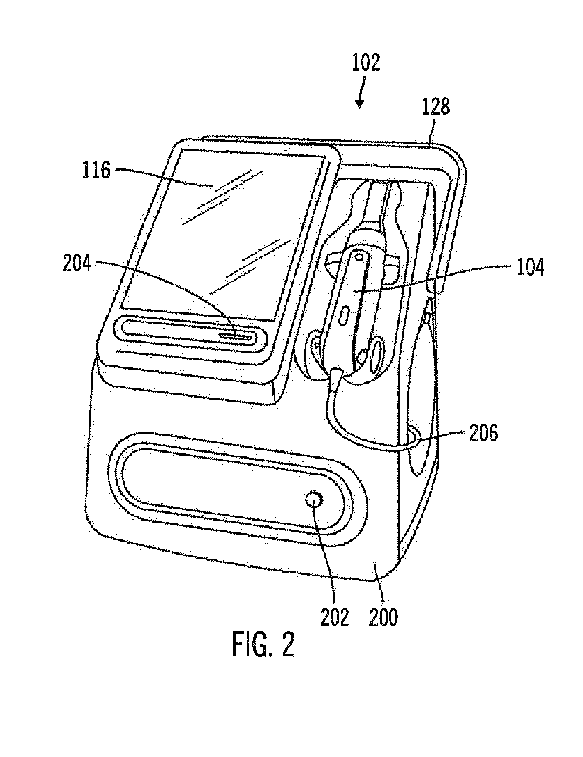

FIG. 2 illustrates a front view of the 3D intra-oral imaging system 102, in accordance with certain embodiments. Certain components of the 3D intra-oral imaging system 102 are enclosed by a hard housing 200 as shown in FIG. 2. The housing 200 is also referred to as a cradle and may house the internal and electronic components of the 3D intra-oral imaging system 102 that are used for data processing. The internal components may include at least the processor 114, the data acquisition and storage hardware 112, the networking hardware 120, and the battery driven power supply 130. The housing 200 has a relatively small footprint for portability.

The surface of the housing 200 is hard and non-porous to facilitate disinfection between uses. The wand 104 is shown placed over the wand storage area. The wand enclosure of the wand protects the optical components of the wand from dust and debris to maintain measurement accuracy. The touchscreen display 116 is shown facing towards the front of the 3D intra-oral imaging system 102. The handle 128 is shown in a position where the handle 128 does not obscure any part of the touchscreen display 116.

In addition to the handle 128, the touchscreen display 116, the wand 104, and the housing 200, the 3D intra-oral imaging system 102 includes a power button 202 that is located on the front face of the 3D intra-oral imaging system 102. The power button 202 may be used to switch the 3D intra-oral imaging system 102 on and off. Additionally, light emitting diode (LED) based indicators 204 may indicate one or more status related to the operational state of the 3D intra-oral imaging system 102. For example, in certain embodiments the LED indicator 204 may be lit when the 3D intra-oral imaging system 102 has been switched on via the power button 202. In certain embodiments, colored LED switches or flashing LED switches may indicate various error or operational states associated with the 3D intra-oral imaging system 102, in addition to or instead of displaying such states on the touchscreen display 116.

In FIG. 2, it can be seen that the wand 104 is connected by a cable 206 to the inside of the housing 200. The cable 206 is used for electrical communications and data transmission between the wand 104 and the data acquisition and storage hardware 112 and other components that are present within the housing 200. The cable 206 also allows the sending of exposure parameters for the camera 108 included in the wand 104. It is important that the cable 206 be light weight and flexible. The properties of the cable 206 may impact the ergonomics and ease of use of the device. Balanced against this is the engineering requirement to isolate and shield a number of high-speed, low-voltage electrical signals communicating to the wand 104 via the cable 206. In certain embodiments, the cable 206 is as light weight and flexible as possible, once engineering limits are considered.

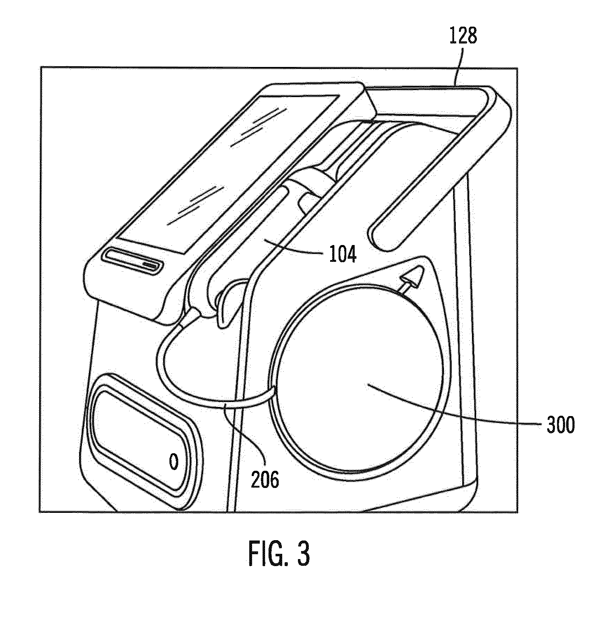

FIG. 3 illustrates a side view of the 3D intra-oral imaging system 102 to show rotations of the handle, 128 in accordance with certain embodiments, where a handle is a part that is designed to be held with one or more hands, and where the handle used for carrying the 3D intra-oral imaging system 102. In certain embodiments, the handle 128 is designed to be attached to the housing 200 via a hinge, such that the handle rotation is restricted to create clearance for a cord wrap mechanism 300 that is used to wrap the cord 206 when the wand 104 is placed on the wand storage area or when the cord 206 is not fully extended. In alternative embodiments, the 3D intra-oral imaging system 102 may have the handle 128 coupled to the housing 200 via mechanism that is different from a hinge. In yet further embodiments, the intra-oral imaging system 102 may have a handle that is integral into the housing 200. For example, the handle may comprise a recessed groove in the housing 200, and the dental practitioner may place his hand at least partially in the recessed groove for carrying the 3D intra-oral imaging system 102 from one location to another. In certain embodiments, the handle may comprise a recessed handle, a retractable handle, a fold down handle, or any other suitable handle for carrying the 3D intra-oral imaging system 102 from one location to another. The weight and dimensions of the 3D intra-oral imaging system 102 are such that a dental practitioner is able to carry the 3D intra-oral imaging system 102 via a handle, such as the exemplary handle 128.

The cord wrap mechanism 300 may be pulled out or pushed in and the excess cord wrapped around a spindle. A latch may be released when the cord wrap mechanism 300 is pulled out and the excess cord may be automatically or manually wrapped around the spindle. In certain embodiments, a static warp reel may comprise the cord wrap mechanism 300, and the static wrap reel may be built into the side of the 3D intra-oral imaging system 102 to store the cord. Thus, in certain embodiments the cord 206 may comprise a stowable cord that is wrapped within the cord wrap mechanism 300.

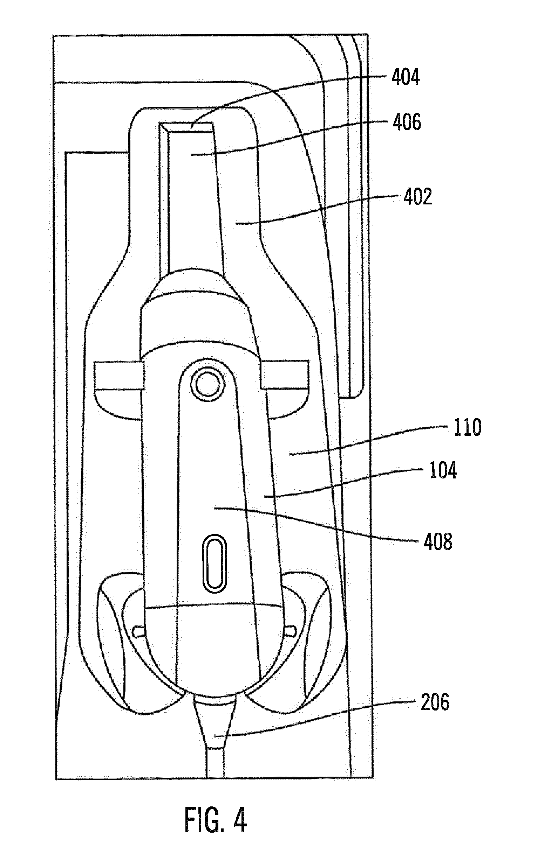

FIG. 4 illustrates the wand 104 and the wand storage area 110 of the 3D intra-oral imaging system 102, in accordance with certain embodiments. In FIG. 4 it can be seen that the wand storage area 110 curves in (reference numeral 402) to protect the tip (reference numeral 404) of the wand, where the tip 404 is located towards the distal end 406 of the wand 104. It may be noted that the base of the wand 104 is towards the proximal end 408 of the wand 104, and the cord 206 is attached to the base of the wand 104.

The tip 404 is the portion of the wand 104 that is inserted into a patient's mouth. In certain embodiments, the maximum length of the tip is restricted to be no more than 115 mm. The maximum height of the windowed end of the tip 404 is restricted to be no more than 20 mm. and the maximum width of the windowed end of the tip is restricted to be no more than 25 mm. Smaller windowed tip sizes are preferred, for reasons of patient comfort and improved intra-oral access. However, imaging accuracy may degrade to unacceptable levels when the windowed tip decreases below certain sizes and in certain embodiments a balance is maintained between the desire for the best possible acceptable imaging accuracy, patient comfort, and intra-oral access. The imaging tip 404 is condensation resistant while operated in a human mouth.

The tip 404 of the 3D intra-oral imaging system 102 may include an optical window made of biocompatible, transparent material that may be either plastic or glass. The window may be mounted into the plastic tip housing such that no sharp corners or edges contact human tissue. In addition, the window properties (i.e., the choice of material and the thickness of material) may be sufficient to prevent fracture inside the oral cavity. In certain embodiments, the disposable tip may not have an optical window and this may allow elimination of saliva contamination on the window surface and the removal of optical reflections.

The size and weight of the wand 104 may important for ergonomic reasons. In certain embodiments, the overall length of the wand 104 is restricted to be less than 300 mm. and the overall width of the wand 104 is restricted to be less than 50 mm. In an exemplary embodiment, the length and width dimensions of the wand 104 are around 200 mm. and 25 mm. respectively. It should be emphasized that the wand tip 404 is designed to be long enough to reach the back teeth of a typical patient.

The weight of the wand 104 is restricted to be no more than 450 grams. In certain embodiments, it is preferred that the weight be below 250 grams. These weights include all internal sensor subsystems and the exterior housing but exclude the cable 206 that connects the wand 104 to the housing 200.

The wand tip 404, used intra-orally, is maintained to be sterile, either through a sterile disposable sheath, cold sterilization immersion, or a removable housing able to withstand repeated steam sterilization.

It should be noted, that the ability of the 3D intra-oral imaging system 102 to produce stable and repeatable interference fringes over the range of temperature and humidity is important for achieving accurate individual fringe images, i.e. a two-dimensional (2D) image of a three dimensional (3D) surface for one camera field of view. The image acquisition and control application 118 may combine multiple individual 2D fringe images to create a 3D point cloud. It should also be noted that the optical components in the wand 104 are kept clean and dust free to maintain measurement accuracy. The electrical current drive to the laser diodes in the optical system is maintained to ensure consistent thermal load and maintain measurement accuracy. The spacing between each laser diode and collimating lens pair is designed to be stable. The spacing between the projector objective lens and the pinhole mask is designed be stable. The phase shift plate and flexure is designed to provide stable and repeatable phase shift values. The spacing between the camera charge coupled device (CCD) and the camera lens of the camera 108 included in the wand 104 is also designed to be stable.

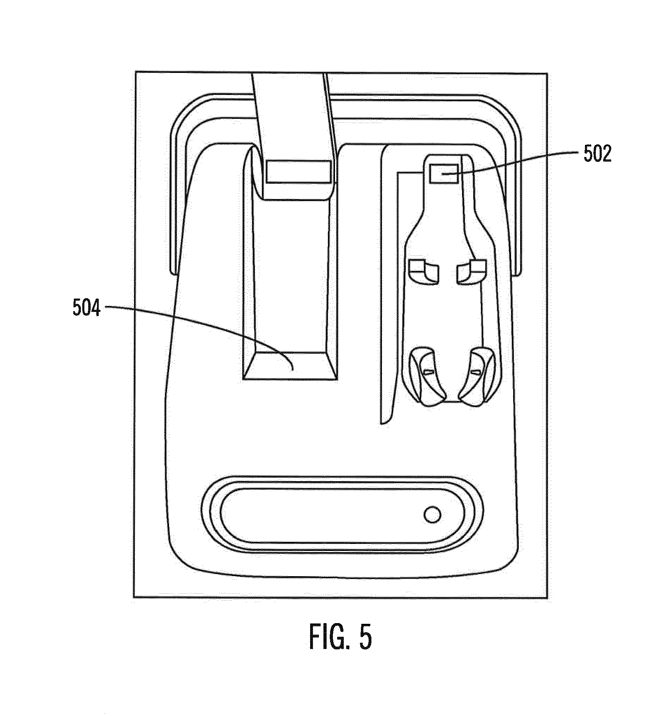

FIG. 5 illustrates a health check artifact 502 of the 3D intra-oral imaging system 102, in accordance with certain embodiments. In certain embodiments the health check artifact 502 is a rectangular area that is about 26 mm. in width and 22 mm. in height. The health check artifact 502 may be configured to determine whether the tip 404 of the wand 104 has been fitted with a clean or otherwise satisfactory disposable mirror or whether a dirty or unsatisfactory mirror is still in use. The health check artifact 502 may also be configured to determine the system measurement accuracy prior to use. In certain embodiments, the health check artifact may be used to determine whether the system measurement accuracy exceeds a certain threshold that may be predetermined. In certain embodiments, measurements of the health check artifact 502 may be used to determine the proper operation and accuracy of the 3D intra-oral imaging system

FIG. 5 also shows that the surface (reference numeral 504) of the housing 200 is smooth and can be cleaned easily. The wand and cradle housings of the 3D intra-oral imaging system 102 may be disinfected between human uses with anti-bacterial, anti-viral, and/or antifungal wipes that are effective at preventing cross contamination when used on hard, non-porous surfaces of the type used on the outer housings 200 of the 3D intra-oral imaging system 102.

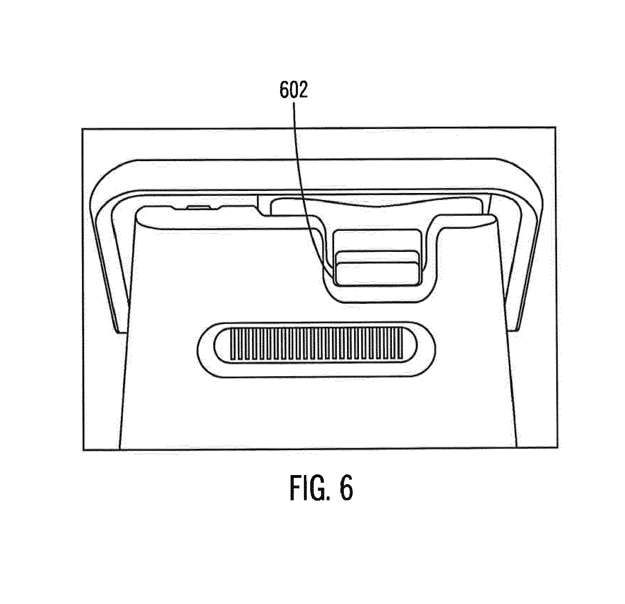

FIG. 6 illustrates a relief cut area 602 for coupling an arm that supports the touchscreen display 116 of the 3D intra-oral imaging system 102, in accordance with certain embodiments. In certain embodiments, the relief cut area 602 for the arm is reduced based on the space needed for an internal hinge mechanism of the arm. The area under the hinge may be increased to allow for easier cleaning and to eliminate the potential for pinch points.

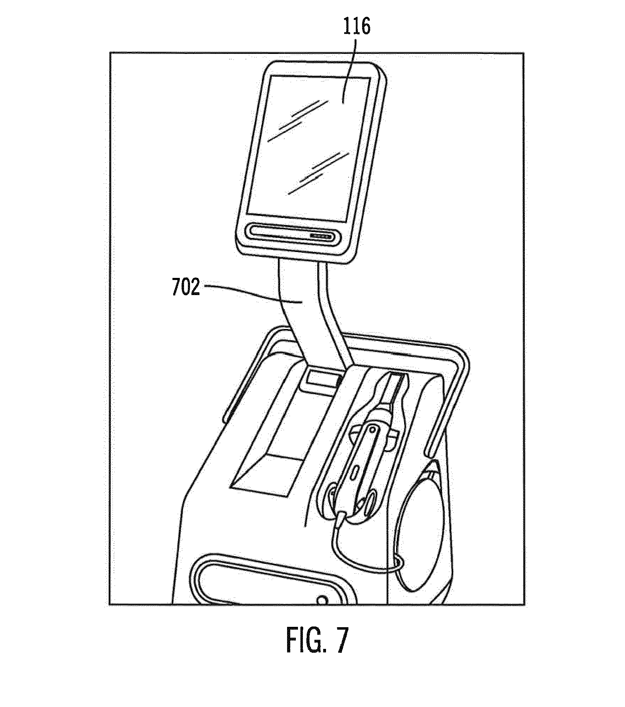

FIG. 7 illustrates an arm 702 that supports the touchscreen display 116 on the housing 200 in the 3D intra-oral imaging system 102, in accordance with certain embodiments. It may seen that the curved shape of the arm 702 makes efficient use of space and causes least impact on the overall height of the 3D intra-oral imaging system 102.

The arm 702 is of a length such that the 3D intra-oral imaging system 102 may be placed on the floor and the arm 702 fully protracted when the intra-oral imaging system 102 is used. In alternative embodiments, the arm 702 may be fully retracted when the intra-oral imaging system 102 is placed on an elevated surface, such as, a chair or a table.

In certain embodiments the arm 702 couples the touchscreen display 116 to the housing 200 in a manner such that the touchscreen display 116 can be tilted and/or rotated.

The arm 702 that supports the touchscreen display 116 is spring counterbalanced such that it takes little force to lift up the touchscreen display 116. However, friction bearings cause the touchscreen display to stay in place in a stable manner when the arm 702 has been extended to lift up the touchscreen display 116.

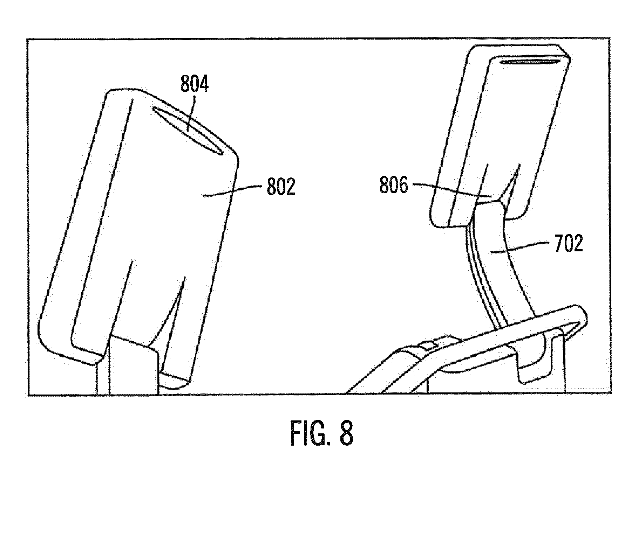

FIG. 8 illustrates various features in a display enclosure 802 for the touchscreen display 116 of the 3D intra-oral imaging system 102, in accordance with certain embodiments. The recessed area (reference numeral 804) on the back of the display enclosure 802 assists the user to pull the touchscreen display 116 out of the stored position into the upright position. Another recessed area (reference numeral 806) along the back of the display enclosure 802 allows the arm 702 to be stored flush to the housing 200.

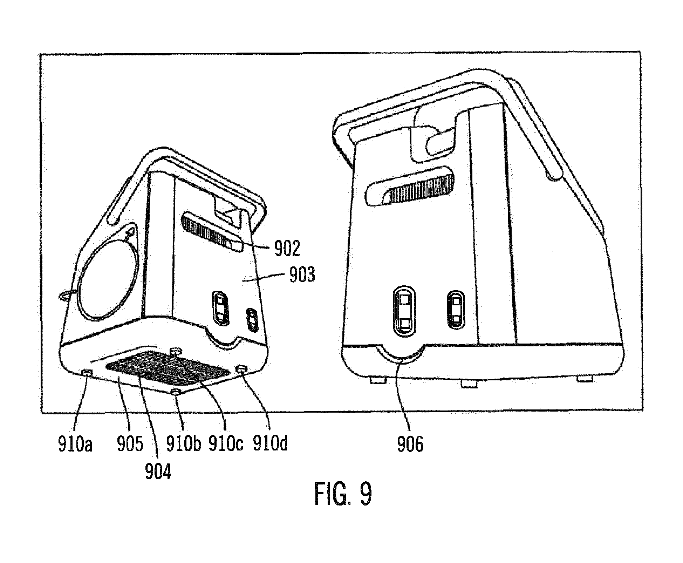

FIG. 9 illustrates venting areas 902, 904 in the 3D intra-oral imaging system 102, in accordance with certain embodiments. The venting area 902 is in a cut out pocket on the back surface 903 of the housing 200, and the venting area 904 is on the bottom surface 905 of the housing 200. The venting areas 902, 904 have vents that facilitate cooling of the 3D intra-oral imaging system 102.

FIG. 9 also shows a handle 906 along the bottom edge of the 3D intra-oral imaging system 102 that allows the user to remove the back panel of the 3D intra-oral imaging system 102 to access a battery that provides uninterrupted power supply to the 3D intra-oral imaging system 102. The battery is a part of the battery driven power supply 130 shown in FIG. 1. The back panel may also need to be removed to perform servicing of the 3D intra-oral imaging system 102 at periodic intervals or to repair internal components located within the housing 200.

The 3D intra-oral imaging system 102 is supported by four pegs 910a, 910b, 910c, 910d. In certain embodiments, the pegs may be made of rubber or other non-slip material that prevents slippage. The pegs 910a, 910b, 910c, 910d are shaped in the form a cylinder or a tapered pin and are mounted to the bottom surface 905 of the 3D intra-oral imaging system 102.

In other embodiments, casters may be substituted for some or all of the pegs. For example, in certain alternative embodiments the two supporting elements 910a, 910b towards the front of the 3D intra-oral imaging system may be pegs and the two supporting elements 910c, 910d towards the rear of the 3D intra-oral imaging system 102 may be comprised of casters, where the casters may comprise small wheels. The casters may provide ease of mobility whereas the pegs may provide resistance to slippage of the 3D intra-oral imaging system 102.

The center of gravity of the 3D intra-oral imaging system 102 is relatively low because of the low weight of the touchscreen display 116 when compared to the components included within the housing 200. As a result, the 3D intra-oral imaging system 102 is stable when placed on a horizontal surface.

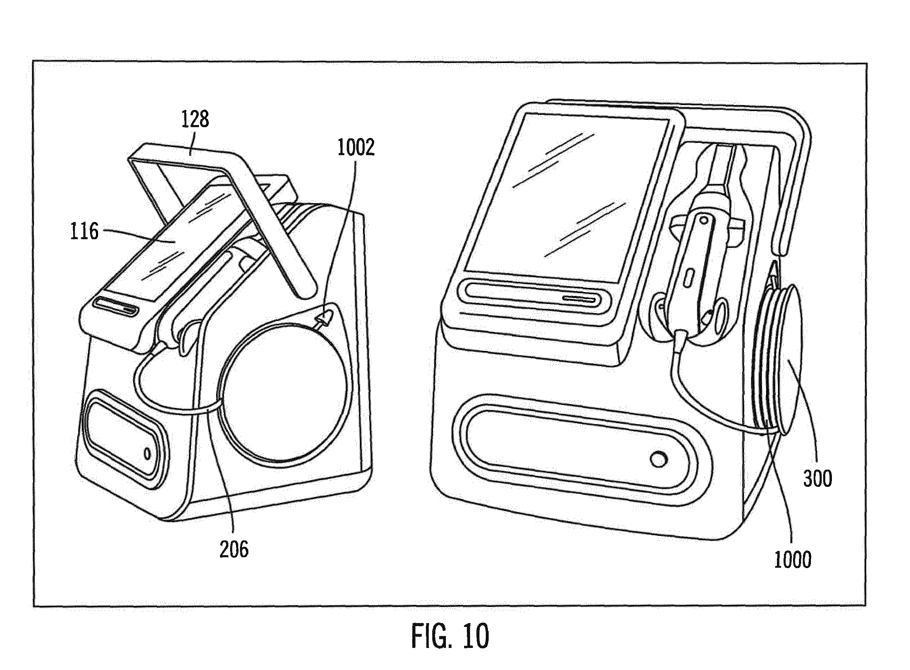

FIG. 10 illustrates how a cord reel 1000 and the handle 128 are implemented the 3D intra-oral imaging system 102, in accordance with certain embodiments. In certain embodiments the cord reel 1000 extends out about 2.5 cm. to allow the user to wrap the cord 206 around the cord wrap mechanism 300.The range of motion of the hinged handle 128 is limited along both directions such that the handle 128 does not touch either the touchscreen display 116 or the cord 206 or the cord warp mechanism 300. A cord strain relief mechanism 1002 is provided on the exit point of the cord from the housing 200.

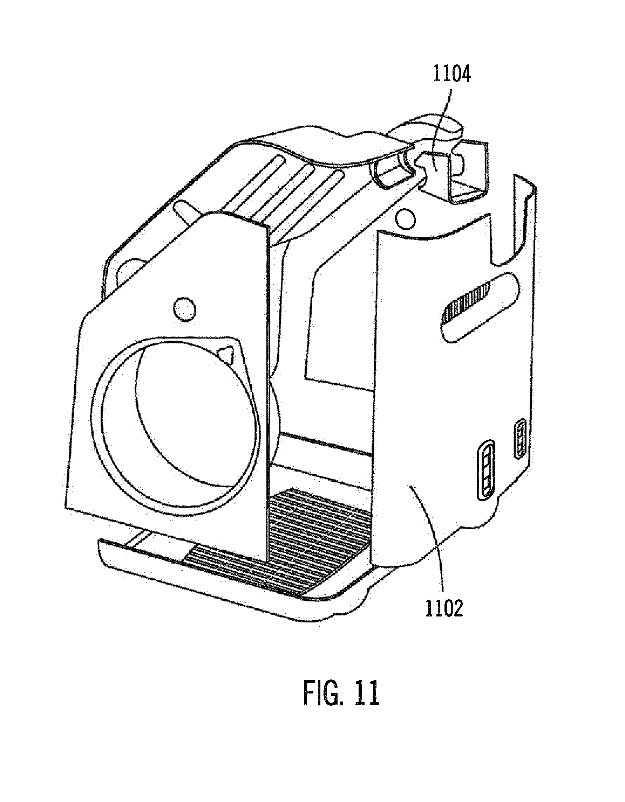

FIG. 11 illustrates at least a back panel 1102 of the housing 200 of the 3D intra-oral imaging system 102, in accordance with certain embodiments. It can be seen from FIG.11 that the split portion (reference numeral 1104) of the front panel of the housing 200 captures the arm 702.

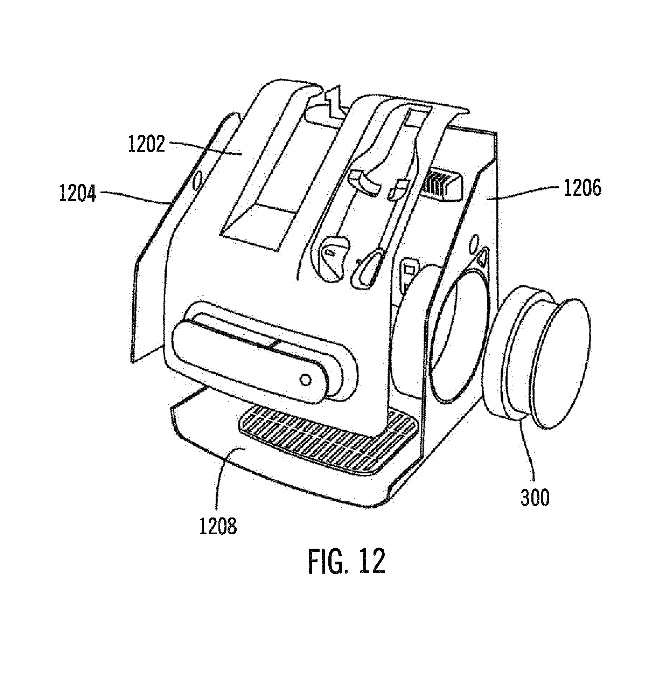

FIG. 12 illustrates at least the front panel 1202, the left panel 1204, the right panel 1206, and the bottom panel 1208 of the 3D intra-oral imaging system 102, in accordance with certain embodiments. The cord reel 300 around which the cord can be wrapped is shown separately in FIG. 12.

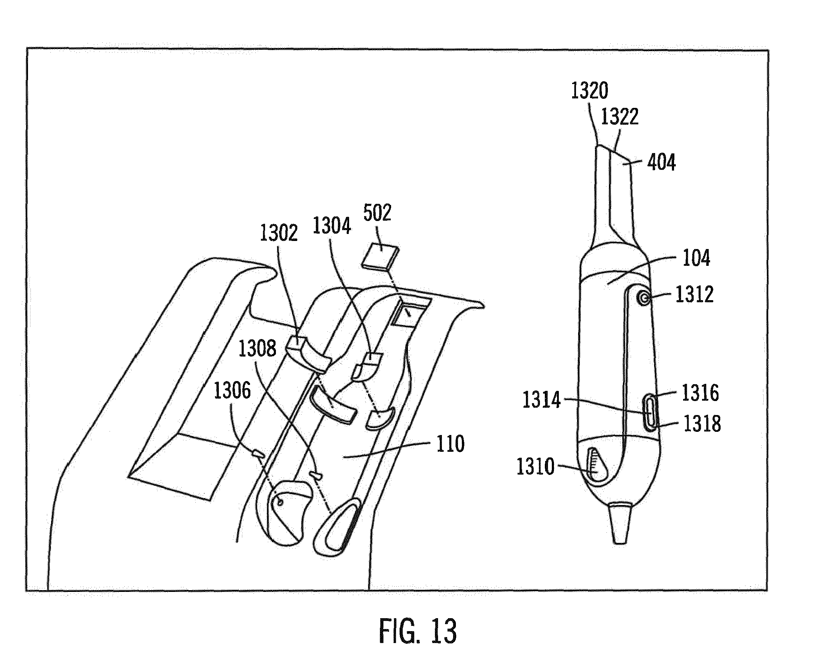

FIG. 13 illustrates how the wand 104 is aligned with respect to the wand storage area 110 of the 3D intra-oral imaging system 102, in accordance with certain embodiments. The two wand attachment mandibles 1302, 1304 allow the wand 104 to stay rigidly fixed and attached to the wand storage area 110. While wand attachment mandibles 1302, 1304 have been shown in FIG. 13, in alternative embodiments the wand attachment mandibles 1302, 1304 may comprise any fasteners that grip the wand 102.

The two wand alignment features 1306, 1308 in the wand storage area 110 allow the wand 104 to be aligned with respect to the wand storage area 110. In certain embodiments, the wand alignment features 1306, 1308 are comprised of rubber nibs. There are two depressions or cutouts corresponding to the two rubber nibs on the wand 104 and the rubber nibs 1306, 1308 engage the two depressions or cutouts. The wand 104 can rotate around the two nibs 1306, 1308 even if the wand 104 is not fully engaged by the mandibles 1302, 1304. In certain embodiments, the two nibs 1306, 1306 may be any suitable projections on the surface of the wand storage area 110.

When the wand 102 is fully engaged with nibs 1306, 1308 and the mandibles 1302, 1304, the tip of the nib is aligned with the health check indicator 502. Additional wand alignment features (reference numeral 1310) on the wand 102 allows further ease of mounting of the wand 104 to the wand storage area 110.

The wand 104 has a round molded area in which there is a first switch 1312 that when pressed by a dental practitioner can start recording of images on and off. There is a second switch that is located 180 degrees across from the first switch in another round molded area (not visible in FIG. 13). The presence of the two switches allow ease of use for acquiring images of the lower and upper arches and also facilitates use by both left and right handed dental practitioners. In certain alternative embodiments, a single switch may be used.

In the oval molded area 1314 there are two keypad buttons 1316, 1318 at the two narrow ends of the oval molded area 1314. A dental practitioner may select and traverse through items from the graphical user interface displayed on the touchscreen display 116 by pressing one or more of the two keypad buttons 1316, 1318 on the oval molded area 1314. Essentially, the oval molded area 1314 includes a forward and a backward keypad button for the graphical user interfaces displayed on the touchscreen display 116. The graphical user interfaces are such that in a mode referred to as a "sequential fully guided mode" a dental practitioner may acquire and manipulate images by pressing the two keypad buttons 1316, 1318 on the oval molded area 1314 of the wand 104 without touching the touchscreen display 116. There is no need to touch the touchscreen except possibly for entering patient information prior to imaging the teeth and /or for final review at the end of the process of imaging the teeth. The two keypad buttons 1316, 1318 and the switch 1312 provide fingertip operating control of the 3D intra-oral imaging system 102.

In certain embodiments, the switch 1312, and the keypad buttons 1316, 1318 may be referred to as controls, and in certain embodiments the recording of images and the navigating of the interfaces of the touchscreen display be achieved via one or more of the controls.

In certain embodiments, the tip 404 of the wand 104 is covered with a disposable molded plastic sheath 1320 that snaps on and off the wand 104. A disposable mirror 1322 is incorporated into the disposable molded plastic sheath 1320. The health check artifact 502 may be used to determine whether the disposable molded plastic sheath 1320 is a new one and whether the disposable mirror 1322 is clean. The housing 200 includes the built-in health check artifact 502 to verify system accuracy. A built-in disposable heater may be used for defogging the disposable mirror 1322.

The health check artifact 502 is activated when the wand 104 is fully engaged by the nibs 1306, 1308 and the mandibles 1302, 1304. The health check artifact 502 may be used to measure three dimensional shapes to determine whether the disposable plastic sheath 1320 on the wand 104 is a new one and whether the disposable mirror 1322 is clean and to verify system measurement accuracy. As a result infection control is improved for the 3D intra-oral imaging system 102.

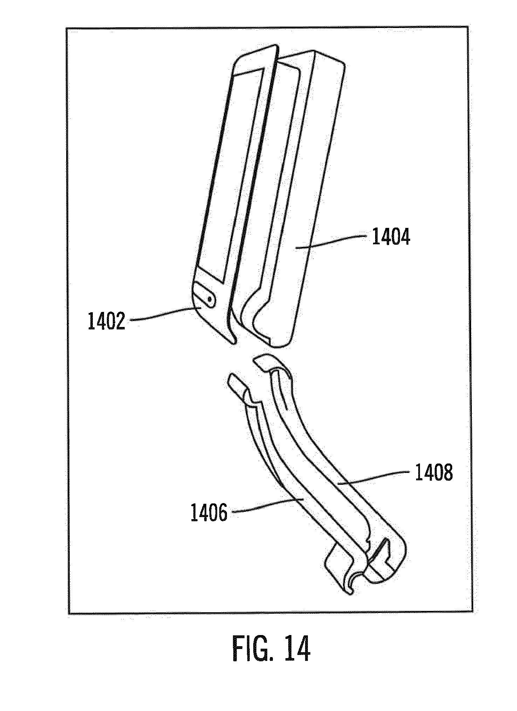

FIG. 14 illustrates how components of the arm 702 and the display enclosure 802 are positioned and oriented in accordance with certain embodiments. The display enclosure 802 is comprised of a front display enclosure 1402 and a back display enclosure 1404 that are coupled to form the display enclosure 802. The front display enclosure 1402 fits over the touchscreen display 116. The arm 702 is a split arm formed from two panels 1406, 1408.

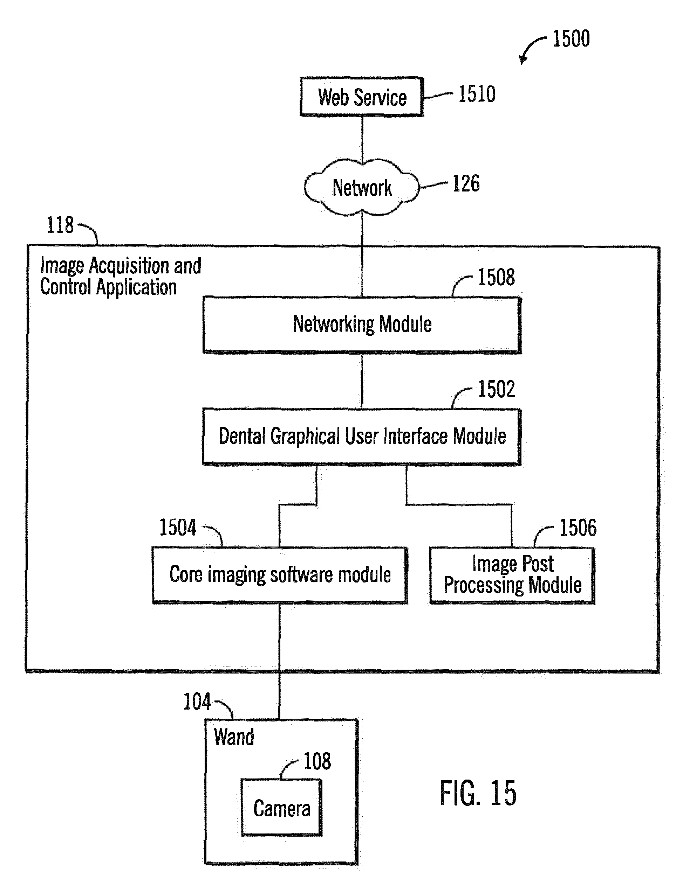

FIG. 15 illustrates a block diagram 1500 of software components that comprise the image acquisition and control application 118 implemented in the 3D intra-oral imaging system 102, in accordance with certain embodiments. In certain embodiments, the image acquisition and control application 118 that executes operations in the processor 114 is comprised of a dental graphical user interface (GUI) module 1502, a core imaging software module 1504, an image post-processing software module 1506, and a networking module 1508.

The dental GUI module 1502 provides interactions to direct the dental practitioner in acquiring the digital impression data for a patient. The dental GUI module 1502 takes input commands from the touchscreen display 116 or commands from the wand 104. After the data has been acquired, the data may be packaged into a data file for upload to a web service 1510 running on computational devices 124a...124n coupled to the network 126.

The core imaging software module 1504 performs data acquisition and display functions. The core imaging software module 1504 interacts with the wand 104 and receives data from the camera 108 as input and produces as output a three-dimensional point cloud representation of the acquired data.

The image post-processing software module 1506 takes as input a three dimensional point cloud representation and produces as output a three dimensional surface for display on the touchscreen display 116.

The networking module 1508 transfers both an encrypted low resolution thumbnail data file (for inspection) and an encrypted high resolution data file (for manufacture) to the web service 1510 via the networking hardware 120. The thumbnail data file may normally be transmitted to the web service 1510 substantially immediately, while the high resolution data file may be transmitted to the web service 1510 at a later time.

It is important that dental practitioners receive indication that the 3D intra-oral imaging system 102 is functioning properly before the device is used to collect digital impression data. A built in, automated "health check" function is provided for this purpose and an indication of health status is presented to the dental practitioner after the built in function completes its test. In certain embodiments, the health check function determines that the camera 108 detects appropriate levels of light from each laser channel when a known, static target is illuminated. The health check function also determines that a 3D intra-oral imager phase shifter generates gray scale images with a sufficiently low intensity ripple when a known, static target is illuminated. Additionally, the health check function also determines that the software generates accurate point clouds containing a specified minimum number of unmasked (i.e., good) points and a specified maximum root mean square fit error when a known, static target is illuminated.

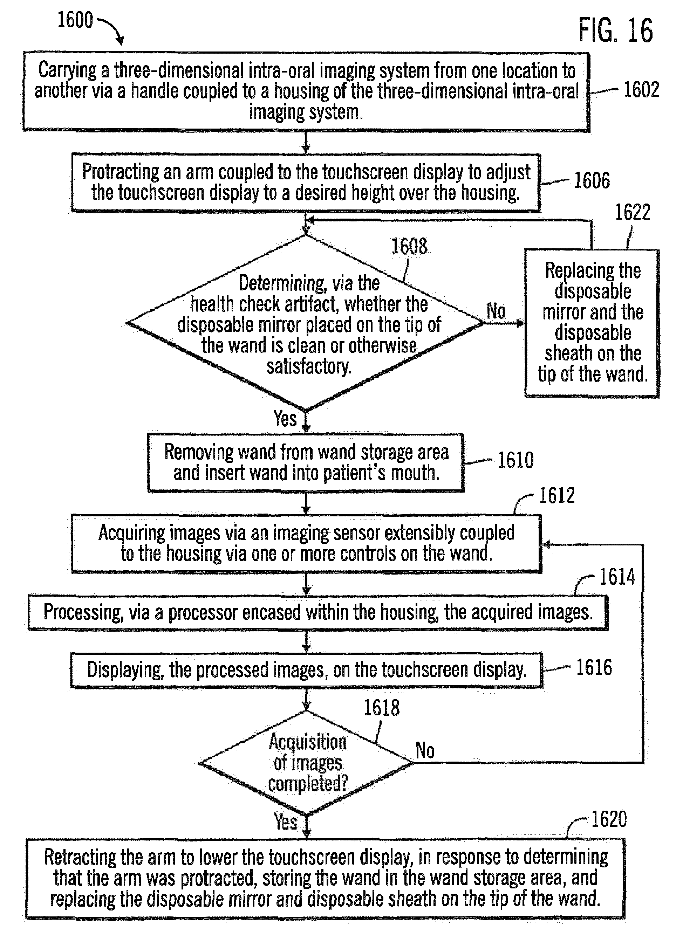

FIG. 16 illustrates a block diagram 1600 of certain operations performed with respect to the 3D intra-oral imaging system 102, in accordance with certain embodiments.

Control starts at block 1602, in which a 3D intra-oral imaging system 102 is carried from one location to another via a handle coupled to the housing 200 of the 3D intra-oral imaging system 102. An arm 702 coupled to the touchscreen display 116 is protracted (at block 1606) to adjust the touchscreen display to a desired height over the housing 200.

Control proceeds to block 1608, in which a determination is made via a health check artifact 502, whether a disposable mirror 1322 placed on the tip 404 of the wand 104 is clean or otherwise satisfactory. If so, then the wand 104 is removed from the wand storage area 110 and inserted into the mouth of a patient (at block 1610). Images are acquired (at block 1612) via an imaging sensor 108 extensibly coupled to the housing 200 by pressing one or more controls (such as, one or more switches 1312 and/or two keypad buttons 1316, 1318) on the wand 104. A processor 114 encased within the housing 200 processes (at block 1614) the acquired images. The processor 114 displays (at block 1616) the processed images on the touchscreen display 116.

Control proceeds to block 1618 in which a determination is made as to whether the acquisition of images has been completed. In response to determining that acquisition of images is completed, control proceeds to block 1620 and the arm 702 is retracted to lower the touchscreen display 116, in response to determining that the arm 702 was protracted. Also, the wand 104 is stored in the wand storage area 110. Furthermore, the disposable mirror 1322 and the disposable sheath 1320 on the tip 404 of the wand 104 are replaced.

If at block 1618 the acquisition of images has not been completed then control returns to block 1612 for continued acquisition of images. Additionally, if at block 1608 a determination is made by the health check artifact 502 that the disposable mirror 1322 placed on the tip of the wand is not clean, then the disposable mirror 1322 and/or the disposable sheath 1320 on the tip of wand are replaced (at block 1622) and control returns to block 1608.

FIGS. 1-16 illustrate certain embodiments in which a 3D intra-oral imaging system 102 is comprised of a processor 114, a housing 200 encasing the processor 114, an imaging sensor 108 extensibly coupled to the housing 200 for capturing images and a touchscreen display 116 to display the images acquired by the imaging sensor 108. An arm 702 is coupled to the housing 200 and supports the touchscreen display 116, wherein the arm 702 is configurable to be retracted and protracted to allow placement of the touchscreen display 116 within a range of heights. A handle 128 of the housing 200 is provided for portability.

Therefore FIGS. 1-16 illustrate certain embodiments, in which the 3D intra-oral imaging system 102 replaces in function, the conventional impression methods used for dentistry and orthodontic applications, including the creation of custom brackets and wires for labial and lingual treatments, the generation of electronic models for patient and doctor reviews, the production of customized aligner systems and lab appliances, etc. A "digital impression" output file generated by the 3D intra-oral imaging system 102 may be transferred electronically, increasing the speed and efficiency of orthodontic and other dentistry treatment. In certain embodiments, the 3D intra-oral imaging system 102 is built out of components that cause the intra-oral imaging system 102 to be relatively smaller and of a lesser weight in comparison to 3D intra-oral imagers that are coupled to a computer and monitor on wheels.

### Additional Details of Embodiments

The operations described in FIGS. 1-16 may be implemented as a method, apparatus or computer program product using techniques to produce software, firmware, hardware, or any combination thereof. Additionally, certain embodiments may take the form of a computer program product embodied in one or more computer readable storage medium(s) having computer readable program code embodied therein.

A computer readable storage medium may include an electronic, magnetic, optical, electromagnetic, infrared, or semiconductor system, apparatus, or device, or any suitable combination of the foregoing. The computer readable storage medium may also comprise an electrical connection having one or more wires, a portable computer diskette or disk, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, etc. A computer readable storage medium may be any tangible medium that can contain, or store a program for use by or in connection with an instruction execution system, apparatus, or device.

Computer program code for carrying out operations for aspects of the present invention may be written in any combination of one or more programming languages.

Features of the present invention are described with reference to flowchart illustrations and/or block diagrams of methods, system and computer program products according to certain embodiments. At least certain operations that may have been illustrated in the figures show certain events occurring in a certain order. In alternative embodiments, certain operations may be performed in a different order, modified or removed. Additionally, operations may be added to the above described logic and still conform to the described embodiments. Further, operations described herein may occur sequentially or certain operations may be processed in parallel. Yet further, operations may be performed by a single processing unit or by distributed processing units. Computer program instructions can implement the blocks of the flowchart. These computer program instructions may be provided to a processor of a computer for execution.

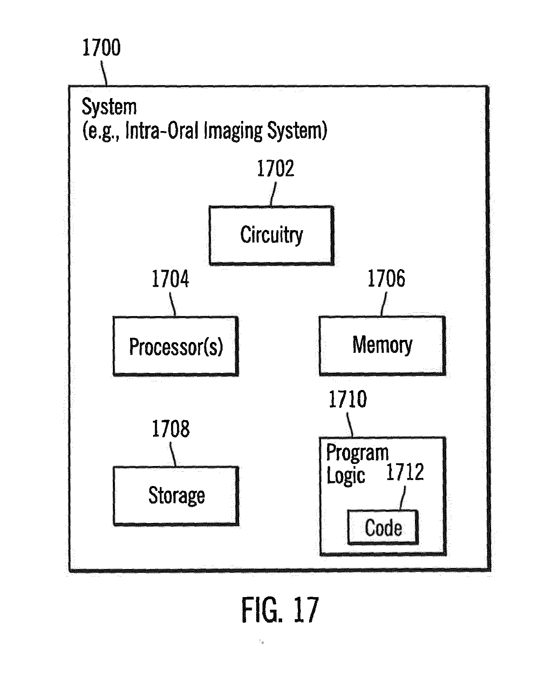

FIG. 17 illustrates a block diagram that shows certain elements that may be included in the 3D intra-oral imaging system 102, in accordance with certain embodiments. The system 1700 may comprise 3D intra-oral imaging system 102 and may include a circuitry 1702 that may in certain embodiments include at least a processor 1704, such as the processor 114. The system 1700 may also include a memory 1706 (e.g., a volatile memory device), and storage 1708. The storage 1708 may include a non-volatile memory device (e.g., EEPROM, ROM, PROM, RAM, DRAM, SRAM, flash, firmware, programmable logic, etc.), magnetic disk drive, optical disk drive, tape drive, etc. The storage 1708 may comprise an internal storage device, an attached storage device and/or a network accessible storage device. The system 1700 may include a program logic 1710 including code 1712 that may be loaded into the memory 1706 and executed by the processor 1704 or circuitry 1702. In certain embodiments, the program logic 1710 including code 1712 maybe stored in the storage 1708. In certain other embodiments, the program logic 1710 may be implemented in the circuitry 1702. Therefore, while FIG. 17 shows the program logic 1710 separately from the other elements, the program logic 1710 may be implemented in the memory 1706 and/or the circuitry 1702.

The terms "an embodiment", "embodiment", "embodiments", "the embodiment", "the embodiments", "one or more embodiments", "some embodiments", and "one embodiment" mean "one or more (but not all) embodiments of the present invention(s)" unless expressly specified otherwise.

The terms "including", "comprising", "having" and variations thereof mean "including but not limited to", unless expressly specified otherwise.

The enumerated listing of items does not imply that any or all of the items are mutually exclusive, unless expressly specified otherwise.

The terms "a", "an" and "the" mean "one or more", unless expressly specified otherwise.

Devices that are in communication with each other need not be in continuous communication with each other, unless expressly specified otherwise. In addition, devices that are in communication with each other may communicate directly or indirectly through one or more intermediaries.

A description of an embodiment with several components in communication with each other does not imply that all such components are required. On the contrary a variety of optional components are described to illustrate the wide variety of possible embodiments.

When a single device or article is described herein, it will be readily apparent that more than one device/article (whether or not they cooperate) may be used in place of a single device/article. Similarly, where more than one device or article is described herein (whether or not they cooperate), it will be readily apparent that a single device/article may be used in place of the more than one device or article or a different number of devices/articles may be used instead of the shown number of devices or programs. The functionality and/or the features of a device may be alternatively embodied by one or more other devices which are not explicitly described as having such functionality/features.

The foregoing description of various embodiments of the invention has been presented for the purposes of illustration and description. It is not intended to be exhaustive or to limit the invention to the precise form disclosed. Many modifications and variations are possible in light of the above teaching.

## Claims

1. A portable three-dimensional intra-oral imaging system, comprising:
a processor;
a housing encasing the processor;
an imaging sensor extensibly coupled to the housing for capturing images;
a touchscreen display to display the images acquired by the imaging sensor;
an arm coupled to the housing and supporting the touchscreen display, wherein the arm is configurable to be retracted and protracted to allow placement of the touchscreen display within a range of heights; and
a handle of the housing for portability.

2. The portable three-dimensional intra-oral imaging system of claim 1, wherein the handle is hinged or integral to the housing.

3. The portable three-dimensional intra-oral imaging system of claim 1 or 2, further comprising:
a wand comprising a proximal end and a distal end, the proximal end extensibly attached to the housing by a stowable cord, the distal end having a tip that holds a disposable mirror;
a wand storage area located on the housing, the wand storage area comprising at least one surface to protect the tip of the wand, wherein the wand storage area engages the wand with one or more fasteners that grip the wand and one or more projections that fit into the wand; and
one or more controls located on the wand to start and stop recording the images and to navigate graphical user interface items displayed on the touchscreen display.

4. The portable three-dimensional intra-oral imaging system of claim 3, wherein the tip is at least partially covered with a disposable sheath, wherein the disposable mirror is placed on or in the tip, wherein the stowable cord is wrapped around a cord wrap mechanism in response to the wand being placed on the wand storage area, and wherein the handle does not touch the stowable cord.

5. The portable three-dimensional intra-oral imaging system of claim 4, further comprising:
a health check artifact located on or in the housing, wherein in response to the wand being fully engaged with the one or more fasteners and the one or more projections, the tip of wand is aligned with the health check artifact, and wherein the health check artifact is configured to determine whether the disposable mirror placed on the tip of the wand is clean or otherwise satisfactory and to determine whether measurement accuracy of the three-dimensional imaging system exceeds a predetermined threshold.

6. The portable three-dimensional intra-oral imaging system of any preceding claim, the housing having a bottom surface with a front end and a back end, the portable three-dimensional intra-oral imaging system further comprising:
two pegs mounted at the bottom surface of the housing towards the front end to provide stability, wherein the plurality of pegs are fabricated out of rubber or other non-slip material to prevent slippage; and
two casters mounted at the bottom surface of the housing towards the back end to facilitate mobility.

7. The portable three-dimensional intra-oral imaging system of any preceding claim, further comprising:
a battery driven power supply enclosed in the housing.

8. The portable three-dimensional intra-oral imaging system of claim 7, the housing further comprising:
a bottom panel with a first set of ventilating holes; and
a back panel with a second set of ventilating holes, wherein the first and the second set of ventilating holes allow circulation of air and dissipation of heat, and wherein at least one panel is removable to allow access to the battery driven power supply enclosed in the housing.

9. The portable three-dimensional intra-oral imaging system of any preceding claim, **characterized in that** the touchscreen display is tiltable, and the arm is rotatable and includes a spring counterbalancing mechanism to lift the touchscreen display, and a friction bearing mechanism to keep the touchscreen display in a stationary position, in response to the touchscreen display being lifted.

10. The portable three-dimensional intra-oral imaging system of any preceding claim, wherein the three-dimensional intra-oral imaging system is not attached to any keyboard or mouse, the portable three-dimensional intra-oral imaging system further comprising:
a hard disk or solid state memory device protectively encased within the housing to store the images acquired by the imaging sensor.

11. The portable three-dimensional intra-oral imaging system of any preceding claim,
wherein the touchscreen display is disposed in a display enclosure, and wherein a recessed area in the display enclosure assists a user to pull the touchscreen display out of a stored position into an upright position; and
wherein another recessed area in the display enclosure allows the arm to be stored flush to the housing.

12. The portable three-dimensional intra-oral imaging system of claim 1 or 2, further comprising:
a wand comprising a proximal end and a distal end, the proximal end extensibly attached to the housing by a retractable cord, the distal end having a tip that holds the imaging sensor;
a wand storage area located on the housing, the wand storage area comprising at least one surface to protect the tip of the wand, wherein the wand storage area engages the wand with fasteners that grip the wand and projections that fit into the wand;
a plurality of pegs supporting the housing; and
a battery driven power supply enclosed in the housing.

13. The three-dimensional intra-oral imaging system of claim 12, wherein the tip is covered with a disposable sheath, and wherein a disposable mirror is placed on the tip, the wand further comprising:
one or more controls located on the wand to start and stop recording the images and to navigate graphical user interface items displayed on the touchscreen display; and
a health check artifact located on or in the housing, wherein in response to the wand being fully engaged with the fasteners and the projections, the tip of wand is aligned with the health check artifact, and wherein the health check artifact is configured to determine whether the disposable mirror placed on the tip of the wand is clean or otherwise satisfactory and to determine whether measurement accuracy of the three-dimensional imaging system exceeds a predetermined threshold.

14. The three-dimensional intra-oral imaging system of claim 13, **characterized in that** the touchscreen display is tiltable and is disposed in a display enclosure, wherein a recessed area in the display enclosure assists a user to pull the touchscreen display out of a stored position into an upright position, and the arm is rotatable and includes a spring counterbalancing mechanism to lift the touchscreen display and a friction bearing mechanism to keep the touchscreen display in a stationary position, in response to the touchscreen display being lifted, and wherein another recessed area of the display enclosure allows the arm to be stored flush to the housing.

15. A method of using the system of any one of claims 1-14, comprising:
carrying the three-dimensional intra-oral imaging system from one location to another via the handle;
protracting the arm to adjust the touchscreen display to a desired height over the housing;
acquiring images via the imaging sensor;
processing, via the processor, the acquired images; and
displaying, via the processor, the processed images on the touchscreen display.

16. A method of using the system of claim 5 or 13, comprising:
carrying the three-dimensional intra-oral imaging system from one location to another via the handle;
protracting the arm to adjust the touchscreen display to a desired height over the housing;
acquiring images via the imaging sensor;
processing, via the processor, the acquired images;
displaying, via the processor, the processed images on the touchscreen display; and
in response to determining that acquisition of images is completed, performing:
retracting the arm to lower the touchscreen display, in response to determining the arm was protracted;
storing the wand in the wand storage area;
determining, via the health check artifact, whether the disposable mirror placed on the tip of the wand is clean or otherwise satisfactory; and
in response to determining that the disposable mirror placed on the tip of the wand is not clean or otherwise satisfactory, replacing the disposable mirror and the disposable sheath on the tip of the wand.
